# FASCICULE DE BREVET EUROPEEN

(11) **EP 0 406 040 B1**
(45) Date de publication et mention de la délivrance du brevet: **18.01.1995**
(21) Numéro de dépôt: 90401522.9
(22) Date de dépôt: 06.06.1990
(51) Int. Cl.: A61F 2/36

(54) **Rotule pour prothèse**
Kugelgelenk für Prothese
Prosthesis ball-joint

(30) Priorité: 28.06.1989 FR 8908598; 21.02.1990 US 482609
(43) Date de publication de la demande: 02.01.1991
(73) Titulaire: LA BIOMECANIQUE INTEGREE, F-91220 Bretigny-sur-Orge (FR)
(72) Inventeur: Bouvet, Jean-Claude, F-91590 La Ferte Alais (FR)
(74) Mandataire: Flavenot, Bernard

(56) Documents cités:
- FR-A- 2 617 706
- GB-A- 1 527 498
- US-A- 4 122 605
- US-A- 4 840 630

## Description

La présente invention concerne les rotules pour prothèses telles que celles qui sont implantées dans le corps humain quand une articulation est détérioriée, par exemple suite à une maladie ou à un accident, notamment celle de la hanche, du genou, etc.

Les chirurgiens sont très souvent amenés à pratiquer des opérations qui consistent à implanter des prothèses, dont les plus courantes sont celles de la hanche et du genou. En effet, suite à des accidents ou des maladies, beaucoup de personnes âgées souffrent, notamment, d'une détérioration de la tête du fémur qu'il est alors nécessaire de remplacer par des moyens comme une prothèse à rotule Cette rotule comporte une partie mâle généralement constituée par une portion de sphère et une partie femelle en forme de cupule complémentaire de la portion de sphère et de même rayon, la partie mâle étant maintenue dans la partie femelle par tous moyens, notamment les tendons et les muscles du patient sur lequel est implantée la prothèse.

Parmi les prothèses de ce type, celles devant plus particulièrement remplacer les articulations de hanche comprennent très schématiquement deux parties, une tête sphérique ou analogue montée par tous moyens sur une patte de fixation destinée à être implantée dans un os long, comme le fémur, et une cupule de forme complémentaire à celle de la tête et apte à être implantée dans l'os du bassin.

Généralement, la réalisation de la patte de fixation ne pose pas de problème, notamment quant à son état de surface ou au choix du matériau la constituant. De même, les moyens actuellement utilisés pour la coopération en rotation de la tête de rotule avec la cupule donnent des résultats tout à fait satisfaisants.

Par contre, la réalisation de la tête de rotule, en l'occurrence la portion de sphère, présente beaucoup plus de difficultés. Les premières têtes ont été réalisées dans des matériaux métalliques. Mais, devant certains problèmes rencontrés, les hommes de l'art ont pensé utiliser des céramiques, c'est-à-dire des matériaux obtenus en noyant des poudres de matières solides dans des adjuvants organiques comme des résines adaptées et en portant ensuite le mélange à haute température.

Actuellement, c'est cette réalisation qui est le plus souvent utilisée, bien que les rotules ainsi obtenues n'aient pas une solidité constante donnant toute satisfaction. Il est en effet souvent constaté que, lorsqu'une telle sphère subit un choc relativement important, par exemple lors d'une chute du patient sur lequel elle est implantée, elle éclate.

Pour essayer de pallier cet inconvénient, il a été réalisé des dispositifs de liaison spéciaux pour associer ces têtes de rotule aux pattes de fixation, mais il est reconnu qu'ils ne présentent pas encore la fiabilité voulue.

Il a même été réalisé une structure particulière de rotule de prothèse comme celle qui est décrite dans le GB-A-1 527 498. La rotule décrite dans ce document comporte deux parties mâle et femelle, la partie mâle comprenant une tête sphérique et la partie femelle comprenant une cupule d'une forme sphérique concave complémentaire de la tête sphérique. La tête sphérique est constituée d'une âme centrale réalisée dans un matériau relativement souple et déformable et d'une pluralités d'inserts de forme sensiblement parallélépipédique réalisés dans un matériau cristallin, les inserts étant enchâssés dans des puits réalisés à la périphérie de l'âme centrale. Cette structure présente des inconvénients: d'une part la surface de frottement entre la tête sphérique et la cupule comporte des discontinuités qui favorisent la production de particules par abrasion, et d'autre part la souplesse du matériau dans lequel est réalisée l'âme centrale entraîne le dessertissage des inserts.

La présente invention a pour but de mettre en oeuvre un procédé pour réaliser une rotule pour prothèse qui pallie les inconvénients mentionnés ci-dessus, tout en alliant les qualités de friction des matériaux comme les céramiques et de solidité du métal, et qui, de plus, ne présente pas de difficultés majeures de réalisation.

Plus précisément, la présente invention a pour objet de mettre en oeuvre un procédé pour réaliser une rotule de prothèse comportant deux parties mâle et femelle, la partie mâle comprenant une tête sphérique et des moyens pour monter fixement ladite tête sphérique sur une patte de fixation, la partie femelle comprenant une cupule d'une forme sphérique concave complémentaire de la tête sphérique,
caractérisé par le fait qu'il consiste à réaliser une âme centrale de forme sphérique dans un matériau métallique et d'un diamètre inférieur à celui requis pour la tête sphérique, et à déposer sous forme vapeur une couche d'un matériau de structure cristalline sur ladite âme centrale jusqu'à obtenir le diamètre requis pour ladite tête sphérique.

D'autres caractéristiques et avantages de la présente invention apparaîtront au cours de la description suivante donnée en regard des dessins annexés à titre illustratif, mais nullement limitatif, dans lesquels :
La figure 1 représente, vu en coupe, un premier mode de réalisation d'une rotule selon l'invention dans le cas d'une prothèse de hanche,
La figure 2 représente, vu en coupe, un deuxième mode de réalisation d'une rotule selon l'invention, également dans le cas d'une prothèse de hanche, et
La figure 3 représente une coupe schématique d'une structure avantageuse pour une rotule selon l'invention.

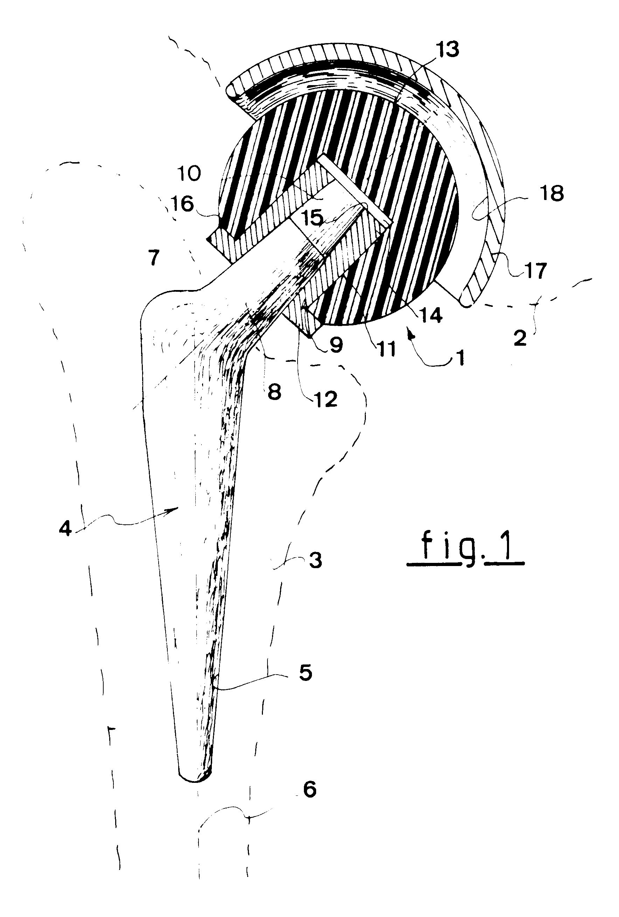

La figure 1 représente, à titre d'exemple, une rotule 1 pour prothèse de hanche.

Cette prothèse de hanche comporte notamment une patte de fixation 4 apte à être fixée dans un orifice 5 pratiqué suivant l'axe 6 du fémur 3.

Les moyens de fixation de cette patte 4 dans le fémur 3 sont connus en eux-mêmes et ne seront pas plus amplement décrits ci-après.

A son extrémité émergente 7, la patte de fixation 4 comprend généralement une partie conique 8 sur laquelle est apte à être emboîté un manchon de liaison 9 comportant un orifice traversant 10 de forme conique complémentaire de la partie conique 8 de la tige 4.

La paroi extérieure 11 de ce manchon de liaison 9 est avantageusement de forme cylindrique et délimitée à une certaine distance de la section la plus faible de la partie conique 8, par exemple, par un épaulement 12 débordant latéralement.

Sur ce manchon est alors enfichée une portion de sphère 13 constituant la partie mâle de la rotule 1. Cette portion de sphère 13 comporte un logement en creux 14 d'une section intérieure au moins égale à celle de la paroi extérieure 11 du manchon de liaison et d'une profondeur au moins égale à la distance séparant le sommet 15 de ce manchon 9 de l'épaulement 12.

De cette façon, la portion de sphère 13 est enfichée sur le manchon 9 jusqu 'à ce que les bords 16 du logement 14 viennent au contact de l'épaulement 12. Elle est alors solidarisée au manchon par tous moyens, par exemple par colle, brasure, etc, selon la nature des matériaux dans lesquels sont réalisés ces deux éléments.

La rotule 1 comporte en outre une partie femelle constituée par une cupule 17 qui est en fait une partie de calotte sphérique concave 18 d'une sphéricité complémentaire de celle de la portion de sphère 13, de telle façon que la portion de sphère puisse épouser cette surface concave 18 dans un emboîtement mâle-femelle parfait.

La technique générale de la rotule décrite ci-dessus pour la réalisation, par exemple, d'une prothèse de hanche, et telle qu'illustrée sur la figure 1, est bien connue en elle-même. Cependant, dans le but d'améliorer la qualité du frottement entre les deux parties de la rotule et d'augmenter la durée de vie d'une telle rotule, et donc de la prothèse la comportant, c'est-à-dire la durée de son implantation, selon la caractéristique principale de l'invention, la portion de sphère 13 est réalisée dans un matériau ayant une structure cristalline, par exemple un monocristal d'alumine comportant différentes quantités de matériaux de dopage comme du fer, du chrome, etc. Pour des applications de haute qualité, il peut même être utilisé un cristal de diamant. La partie femelle de la rotule constituée par la cupule 17 peut être réalisée dans une matière plastique apte à être implantée dans le corps humain, par exemple l'une des matières bien connues couramment utilisées dans ce domaine.

Les monocristaux utilisés pour la réalisation des têtes sphériques 13 sont relativement faciles à obtenir, notamment avec un matériau à base d'alumine, par croissance à partir de germes de ces monocristaux, par exemple par la méthode Czochralski.

Les expérimentations faites sur de telles rotules ont montré que ce type de réalisation donne toute satisfaction, notamment quant à la finition et à la solidité, et ont prouvé que les prothèses ainsi réalisées sont beaucoup plus fiables que celles de l'art antérieur.

Les monocristaux utilisés sont du type saphir, rubis, etc., qui, il faut le reconnaître, sont des matériaux relativement difficiles à usiner, notamment pour réaliser le logement en creux 14 dans la tête sphérique 13 illustré sur la figure 1. En effet, cet usinage "en creux" nécessite l'utilisation d'un outillage adapté de très grande qualité et rend difficile le contrôle de l'état de la surface intérieure du logement car cette surface est difficilement accessible aux appareils qui peuvent effectuer un tel contrôle.

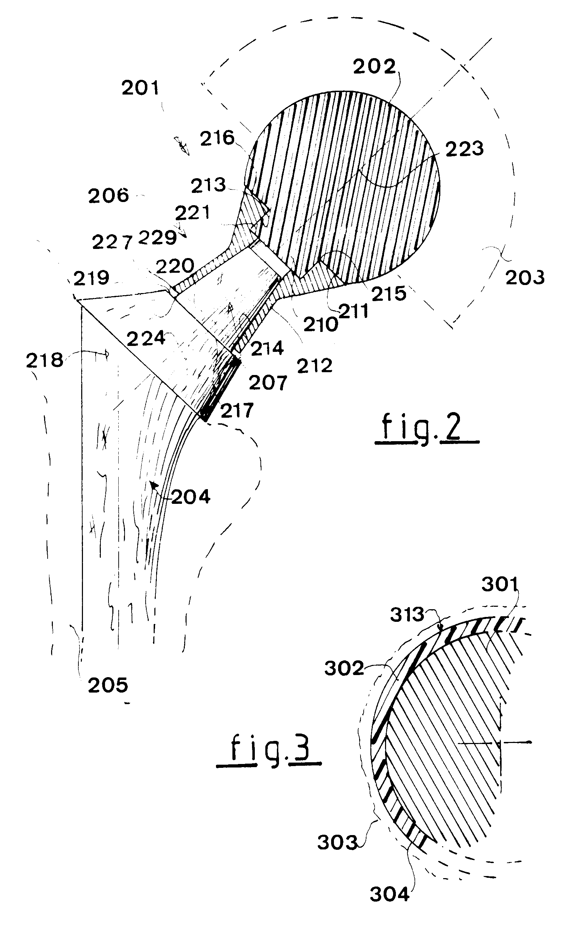

La réalisation illustrée sur la figure 2 permet de pallier ces inconvénients en présentant une structure de tête sphérique qui soit d'une réalisation plus aisée que celle des prothèses de l'art antérieur, particulièrement avantageuse dans le cas d'une tête sphérique en matériau cristallin, et qui permette de contrôler plus facilement l'état des surfaces usinées, dans le but notamment d'assurer aux prothèses une fiabilité de fixation maximale pour éviter des interventions chirurgicales trop fréquentes.

La figure 2 représente un mode de réalisation d'une tête sphérique 202 plus particulièrement conçue pour une prothèse de hanche 201 présentant les avantages mentionnés ci-dessus. Cette tête sphérique 202 est apte à coopérer en rotation dans une cupule ou un cotyle 203. La prothèse 201 comporte en outre une patte de fixation 204 apte à être implantée dans le canal médullaire d'un os long 205 tel qu'un fémur, et des moyens de liaison 206 de la tête sphérique 202 avec une extrémité 207 de la patte 204.

Les moyens de liaison 206 comportent une partie en saillie 210 solidaire d'un méplat 211 réalisé sur la tête sphérique 202 et un manchon 212 comportant deux percées 213 et 214. Une première percée 213 possède une section complémentaire de la partie en saillie 210 qui est enfichée dans cette première percée 213 et la seconde percée 214 a une section sensiblement complémentaire de l'extrémité 207 de la patte de fixation 204 qui est, pour sa part, enfichée dans cette seconde percée.

Dans un mode réalisation avantageux, la partie en saillie 210 a une section inférieure au diamètre du méplat 211 de façon à définir, sur ce méplat, une surface portante sur laquelle pourra venir s'épauler une partie du manchon 212 comme décrit ci-après. Toujours dans le but d'obtenir une structure facilement réalisable par usinage, la partie en saillie 210 et la première percée 213 sont avantageusement de forme cylindrique de révolution.

Pour permettre à la tête sphérique d'absorber la plus grande quantité possible des efforts auxquels la prothèse implantée dans un corps humain est généralement soumise, avant qu'ils ne soient transmis au cotyle, l'ouverture extérieure 215 de la première percée 213 est délimitée par un épaulement externe 216 en forme de couronne circulaire d'un diamètre extérieur sensiblement inférieur ou égal à celui du méplat 211. De cette façon, la tête sphérique prend bien appui sur le manchon 212 par une première surface relativement grande.

Dans le mode de réalisation avantageux illustré, l'extrémité 207 de la patte de fixation 204 est usinée en forme de tronc de cône de révolution dont la grande base 217 définit la surface de liaison entre le corps 218 de la patte et l'extrémité 207 elle-même.

Dans ce cas, la seconde percée 214 est également de forme sensiblement tronconique de révolution mais d'un angle au sommet légèrement inférieur à celui du tronc de cône défissant l'extrémité 207, tout en ayant sa grande base 219 d'une section égale à l'une des sections de cette extrémité de la patte de fixation, par exemple une section au voisinage de celle qui est indiquée en trait interrompus 220 sur la figure 2.

Il a été mentionné ci-avant que la tête sphérique prenait appui sur un premier épaulement 216 du manchon. Afin de mieux encore répartir les efforts exercés sur la prothèse, la surface portante de la tête de prothèse a été augmentée en réalisant, dans le fond de la première percée 213, un épaulement rentrant 221 situé à une distance de l'ouverture extérieure 215 de la première percée égale à la hauteur de la partie en saillie 210.

Pour rendre la réalisation mécanique d'un tel manchon la plus aisée possible, il est avantageux que les fonds, respectivement des deux première 213 et seconde 214 percées, soient confondus, comme illustré sur la figure 1, ainsi que les axes de révolution 223, 224 de ces deux percées. De plus, la surface latérale tronconique de la seconde percée est réalisée de façon que le diamètre de sa petite base soit inférieur à celui de la première percée. La différence des sections forme ainsi directement lors de l'usinage l'épaulement rentrant 221 défini ci-dessus.

Comme mentionné ci-avant, la partie en saillie 210 est solidaire de la tête sphérique 202. Cependant, l'un des moyens avantageux pour obtenir cette solidarisation est de réaliser la tête sphérique et la partie en saillie dans la masse d'un même bloc de matériau, par exemple un monocristal tel que du rubis, du saphir, etc.

La partie mâle de rotule de prothèse telle que décrite ci-dessus et illustrée sur la figure 2 se monte de la façon suivante.

Dès que les trois éléments - tête sphérique, manchon, patte de fixation sont réalisés, la tête sphérique 202 avec sa partie en saillie 210 est montée en coopération avec le manchon 212 de façon à plonger, par cette partie en saillie, dans la première percée 213 du manchon avec lequel elle est solidarisée par tous moyens, notamment par collage, soudage, brasage, selon la nature des matériaux constituant respectivement ces deux éléments.

L'extrémité 207 de la patte de fixation 204 est alors emmanchée dans la seconde percée 214 du manchon Jusqu 'à ce que le bord de la grande base 219 de cette seconde percée vienne au contact de la surface du tronc de cône définissant l'extrémité de la patte. Par un effort supplémentaire d'enfoncement, la paroi du manchon se déforme au niveau de sa seconde percée de façon que la conicité de cette dernière devienne sensiblement égale à celle de l'extrémité de la patte de fixation, la paroi interne de la percée épousant alors parfaitement la surface de l'extrémité 207 de la patte de fixation. Ceci permet de bien solidariser le manchon et la tête sphérique avec la patte de fixation, par un effet de pincement essentiellement dû à l'élasticité du matériau utilisé pour la réalisation, notamment, du manchon.

La structure de tête de prothèse telle que décrite ci-dessus présente de multiples avantages. Il est en effet plus facile d'usiner à l'aide d'outils tels que des meules au diamant un bloc de matériau d'une grande dureté pour obtenir des parties en saillie même de faibles dimensions, que de l'usiner avec des mèches pour y réaliser des logements. De plus, le fait que toutes les surfaces d'usinage de la têtes phérique et de la partie en saillie soient externes rend le contrôle de leurs défauts et qualités beaucoup plus aisé que celui de surfaces en creux.

Enfin, cette structure n'oblige pas à effectuer des percées dans la masse de la sphère et contribue ainsi à limiter l'existence de points de faiblesse de la tête de prothèse, améliorant d'autant la fiabilité d'un tel produit.

La structure de tête de prothèse décrite ci-dessus peut cependant comporter un perfectionnement. Ce perfectionnement est constitué par un épaulement 227 situé au niveau de la grande base 217 de l'extrémité tronconique 207 de la patte 204. Cet épaulement va pouvoir constituer une butée pour limiter l'enfoncement de l'extrémité 207 dans la seconde percée 214 du manchon 212. Cette limitation évite ainsi que de trop grands efforts radiaux soient appliqués à la paroi de cette percée 214 et confère donc au manchon une durée de vie plus importante.

Bien entendu, dans ce cas, le manchon sera conçu, notamment en fonction du matériau dans lequel il est réalisé, pour donner à la paroi de sa seconde percée 214 une épaisseur lui conférant une élasticité de déformation suffisante pour que, d'une part, la couronne circulaire 229 entourant la grande base 219 de cette seconde percée vienne buter contre l'épaulement 227 et que, d'autre part, cette paroi puisse exercer une force de pincement d'intensité suffisante pour obtenir une bonne solidarisation du manchon avec l'extrémité tronconique de la patte.

Dans les deux modes de réalisation décrits et illustrés ci-dessus, la tête sphérique est réalisée dans un bloc de matériau cristallin, soit un monocristal. Cependant, il est bien évident que, pour obtenir une telle portion de sphère, il faut utiliser, à la base, un monocristal assez gros pour pouvoir être travaillé et usiné jusqu'à la forme finale cherchée. Même si, dans certains cas, par exemple pour une prothèse de genou, la forme sphérique n'est nécessaire que sur une faible partie de la prothèse, en général, le rayon de la sphère est relativement grand et la quantité de produit de base est toujours importante. Pour pallier cet inconvénient qui entraîne essentiellement un coût de revient important, la présente invention a aussi pour but de réaliser une structure de rotule qui permette de réduire le coût de revient de la prothèse.

Dans ce cas, comme illustré sur la figure 3, la tête sphérique 313 est essentiellement constituée en deux parties 301 et 302. La partie 301 constitue une âme centrale en un matériau rigide, par exemple métallique tel que de l'acier, un alliage à base de titane, etc, qui est déjà usinée pour avoir une forme sensiblement sphérique, mais avec un diamètre inférieur à celui que doit avoir la tête sphérique dans sa forme définitive.

Sur cette âme centrale est ensuite déposée une couche 302 d'un monocristal, par exemple par la technique des dépôts physiques en phase vapeur connus par les techniciens sous le sigle P.V.D. Ce type de dépôt est obtenu, soit par évaporation simple, la vapeur se condensant sur le support, en l'occurrence la surface sphérique de l'âme centrale 301, soit par pulvérisation cathodique, c'est-à-dire le bombardement du matériau cristallin par des ions du gaz support de la décharge électrique, la phase vapeur se condensant ensuite sur l'âme 301.

Au moins un autre procédé peut être utilisé pour déposer la couche 302 de monocristal sur l'âme centrale 301, celui de la technique des dépôts chimiques en phase vapeur connue par les techniciens sous le sigle C.V.D. Ce procédé consiste schématiquement à réaliser un dépôt à partir d'une réaction chimique entre le composé volatil à déposer et le substrat ou support.

Dans le cas présent, on utilise donc le procédé le plus adapté en fonction du matériau cristallin utilisé et du matériau constituant l'âme centrale 301.

Le dépôt est effectué jusqu 'à une épaisseur 303 supérieure à celle 304 qui est nécessaire pour obtenir le diamètre définitif de la tête sphérique, la couche 302 étant alors usinée pour lui donner la forme et les dimensions voulues.

Cette réalisation est avantageuse car elle ne nécessite que peu de quantité de matériau cristallin et contribue ainsi à la réduction du coût de revient d'une telle prothèse.

## Revendications

1. Procédé pour réaliser une rotule (1) de prothèse comportant deux parties mâle et femelle, la partie mâle comprenant une tête sphérique (13,202,313) et des moyens (9,10,11,206) pour monter fixement ladite tête sphérique sur une patte de fixation (4,204), la partie femelle comprenant une cupule (17) d'une forme sphérique concave (18) complémentaire de la tête sphérique, caractérisé par le fait qu'il consiste à réaliser une âme centrale
(301) de forme sphérique dans un matériau métallique et d'un diamètre inférieur à celui requis pour la tête sphérique, et à déposer sous forme vapeur une couche (302) d'un matériau de structure cristalline sur ladite âme centrale jusqu'à obtenir le diamètre requis pour ladite tête sphérique.

2. Procédé selon la revendication 1, caractérisé par le fait que ledit matériau ayant une structure cristalline est un monocristal.

3. Procédé selon la revendication 2, caractérisé par le fait que ledit monocristal est à base d'alumine cristallisée.

4. Procédé selon la revendication 2, caractérisé par le fait que ledit monocristal est choisi parmi l'un des cristaux suivants: rubis, saphir, diamant.

5. Rotule de prothèse réalisée par la mise en oeuvre du procédé selon l'une des revendications 1 à 4, comportant deux parties mâle et femelle, la partie mâle comprenant une tête sphérique (13,202,313) et des moyens (9,10,11,206) pour monter fixement ladite tête sphérique sur une patte de fixation (4,204), la partie femelle comprenant une cupule (17) d'une forme sphérique concave (18) complémentaire de la tête sphérique, ladite tête sphérique (13,202,313) comprenant une âme centrale (301) de forme sphérique réalisée dans un matériau métallique et d'un diamètre inférieur à celui requis pour la tête sphérique, et une couche (302) d'un matériau de structure cristalline déposé sous forme vapeur sur ladite âme centrale jusqu'à obtenir le diamètre requis pour ladite tête sphérique.

## Claims

1. A method of making a ball-and-socket joint (1) for a prosthesis, the joint comprising a male portion and a female portion, the male portion comprising a spherical head (13, 202, 313) and means (9, 10, 11, 206) for fixing said spherical head on a connection peg (4, 204), the female portion comprising a cup (17) having a concave spherical shape (18) complementary to the spherical head, the method being characterized by the fact that it consists in making a spherically shaped central core (301) out of a metal and having a diameter smaller than that required for the spherical head, and in depositing in vapor form a layer (302) of crystal structure material on said central core until the diameter required for said spherical head is obtained.

2. A method according to claim 1, characterized by the fact that said crystal structure material is a monocrystal.

3. A method according to claim 2, characterized by the fact that said is based on crystallized alumina.

4. A method according to claim 2, characterized by the fact that said monocrystal is selected from one of the following crystals: ruby, sapphire, diamond.

5. A ball-and-socket joint for a prosthesis made by implementing the method according to any one of claims 1 to 4, the joint comprising a male portion and a female portion, the male portion comprising a spherical head (13, 202, 313) and means (9, 10, 11, 206) for fixing said spherical head on a connection peg (4, 204), the female portion comprising a cup (17) having a concave spherical shape (18) complementary to the spherical head, said spherical head (13, 202, 313) comprising a spherically shaped central core (301) made out of a metal and having a diameter smaller than that required for the spherical head, and a layer (302) of crystal structure material deposited in the form of vapor on said central core until the diameter required for said spherical head is obtained.

## Patentansprüche

1. Verfahren zum Herstellen einer Prothesenkugel (1) mit einem Einfügeteil und einem Aufnahmeteil, wobei das Einfügeteil einen sphärischen Kopf (13,202,313) und Mittel (9,10,11,206) zum Befestigen des sphärischen Kopfes an einer Befestigungslasche (4,204) aufweist, während das Aufnahmeteil eine Pfanne (17) in konkavsphärischer Form (18) komplementär zu dem sphärischen Kopf umfaßt, dadurch gekennzeichnet, daß es aus der Herstellung eines zentralen Kerns (301) sphärischer Form aus einem metallischen Material und einem Durchmesser, der kleiner ist als derjenige, der für den sphärischen Kopf erforderlich ist und dem Aufbringen aus der Dampfphase einer Schicht (302) eines Materials kristalliner Struktur auf den zentralen Kern bis zum Erhalten des für den sphärischen Kopf erforderlichen Durchmessers besteht.

2. Verfahren nach Anspruch 1, dadurch gekennzeichnet, daß das Material mit kristalliner Struktur ein Monokristall ist.

3. Verfahren nach Anspruch 2, dadurch gekennzeichnet, daß der Monokristall auf Basis von kristallisiertem Aluminium ist.

4. Verfahren nach Anspruch 2, dadurch gekennzeichnet, daß der Monokristall unter den folgenden Kristallen ausgewählt ist: Rubin, Saphir, Diamant.

5. Prothesenkugel, hergestellt nach dem Verfahren gemäß einem der Ansprüche 1 bis 4, umfaßend eine Einsteckpartie und eine Aufnahmepartie, wobei die Einsteckpartie einen sphärischen Kopf (13,202,313) und Mittel (9,10,11,206) zum Befestigen des sphärischen Kopfes auf einer Befestigungskralle (4,204) umfaßt, während die Aufnahmepartie eine Pfanne (17) konkavsphärischer Form (18) komplementär zu dem sphärischen Kopf umfaßt, welcher sphärische Kopf (13,202,313) einen zentralen Kern (301) sphärischer Form umfaßt, hergestellt aus einem metallischen Material und mit einem Durchmesser, der kleiner ist als jener, der für den sphärischen Kopf erforderlich ist und eine Schicht (302) eines Materials kristalliner Struktur, aufgebracht aus der Dampfphase auf den zentralen Kern bis zum Erreichen des für den sphärischen Kopf erforderlichen Durchmessers.
